# EUROPEAN PATENT APPLICATION

(11) **EP 2 508 886 A2**
(43) Date of publication of application: **10.10.2012**
(21) Application number: 12167621.7
(22) Date of filing: 02.06.2008
(51) Int. Cl.: G01N 33/564

(54) **Means and methods for classifying samples of multiple sclerosis patients**

(30) Priority: 01.06.2007 EP 07109468
(62) Divisional of application: 10169971.8
(71) Applicant: Vereniging VU-Windesheim Vereniging voor Christelijk Hoger Onderwijs, Wetenschappelijk Onderzoek en Patiëntenzorg, 1081 HV Amsterdam (NL)
(72) Inventor: van Baarsen, Elisabeth Geertruida Maria, 1135 AN Edam (NL); Stahlecker-Voslamber, Saskia, 2318 MK Leiden (NL); Verweij, Cornelis Lammert, 1391 CD Abcoude (NL); Polman, Chris Hubert, 2051 HE Overveen (NL)
(74) Representative: Jansen, Cornelis Marinus

(57) **Abstract**

The invention in one aspect provides a method for classifying cells from a human individual said method comprising: providing a sample comprising cells from said individual that are typically responsive to exposure to a type I interferon; determining a level of activity of a pathway that is modulated by type 1 interferon; and classifying said cells on the basis of the determined level of activity. Cells present in said sample are preferably cultured said cells in the presence of a type I interferon prior to determining a level of activity of said pathway. This is activity is preferably compared to the activity of said pathway in cells in said sample prior to said culture. Preferably the sample is from an individual that is not treated with a type I interferon prior to collecting said sample. Preferably the method is used to determine, prior to initiating treatment with a type I interferon, whether said individual is likely to be a good, a normal or a poor responder to the contemplated treatment.

## Description

Interferons (IFNs) are natural proteins produced by the cells of the immune system in response to challenges by foreign agents such as viruses, bacteria, parasites and tumor cells. Today, interferons are approved for treatment of malignancies such as hairy cell leukemia, malignant melanoma, and AIDS-related Kaposi's sarcoma; chronic hepatitis B and C; multiple sclerosis; condylomata acuminate, genital and perianal warts caused by infections with human papillomavirus (HPV); chronic granulomatous disease; renal cell carcinoma (RRC) and severe, malignant osteopetrosis. Clinical trials are ongoing or have been finished to show a clinical benefit of interferon-comprising treatment for other malignancies, virus-mediated diseases and autoimmune-related diseases such as rheumatoid arthritis.

Multiple sclerosis (MS) is a common inflammatory disease of the central nervous system characterized by progressive neurological dysfunction. The disease has a heterogeneous nature, which is reflected in the clinical presentation, ranging from mild to severe demyelinating disease. No curative therapy is currently available, and the majority of affected individuals are ultimately disabled (1).

IFNs were the first agents to show clinical efficacy in Relapsing Remitting MS (RRMS). Interferon beta (IFNbeta) decreases clinical relapses, reduce brain disease activity, and possibly slow progression of disability. However, therapy is associated with a number of adverse reactions, including flu-like symptoms and transient laboratory abnormalities. Moreover, the response to IFNbeta is partial, i.e. disease activity is suppressed by only about one third (2). Clinical experience suggests that there are IFN 'responders' as well as 'non responders' (3;4). In the absence of predictive biomarkers the question remains who will respond to therapy and who to treat when inconvenience and costs are significant.

Part of the unresponsiveness to IFNbeta can be explained by immunogenicity. Antibodies that develop in response to IFN treatment have been characterized as binding or neutralizing antibodies. However, since not all patients develop neutralizing antibodies (Nabs), and, if induced, Nabs appear over time (5-8), other mechanisms are likely to be involved to explain unresponsiveness.

In normal physiology IFNs produce their biological effects by binding to multi-subunit receptors IFNAR-1 and -2 on the cell surface, thereby initiating a complex cascade of intracellular secondary messengers that emerge in two divergent pathways. One pathway leads to activation of the transcription factor ISGF3 (IFN-stimulated gene factor 3), a complex of phosphorylated Signal Transducer and Activator of Transcription (STAT) 2 with STAT1 and IFN regulatory factor 9 (IRF-9; p48) that binds to the IFN-stimulated response element (ISRE) present in multiple genes (9;10). The other pathway involves STAT2/1 and STAT2/3 heterodimers and STAT1 homodimer (IFNalpha-activated factor, AAF), which bind to the IFN gamma-activated sequence (GAS) response element (10-13). Ultimately, the IFN-induced activation of ISRE and GAS enhancer elements turns on a wide variety of genes (14) leading to specific transcriptional changes.

There exists a clear need for the development of a biomarker that allows classifying cells according to a probability to respond to type I interferon.

The present invention provides a method for classifying cells from a human individual said method comprising providing a sample comprising cells from said individual that are typically responsive to exposure to a type I interferon, determining a level of activity of a pathway that is modulated by type 1 interferon, and classifying said cells on the basis of the determined level of activity. It is preferred that said pathway comprises an activation pathway, whereby said activation pathway comprises the transcriptional activation of genes. An important application of the present invention is to determine whether treatment of said individual with a type I interferon is likely to be successful. An individual that has cells that are classified as poor responders is likely not respond well to treatment with a type I interferon.

A preferred method according to the invention further comprises culturing said cells in the presence of a type I interferon prior to determining a level of activity of said pathway.

In a preferred embodiment, said individual suffers from, or is at risk of suffering from, an autoimmune disease. The term autoimmune disease refers to a disease that is characterized by an immune response against an antigen that is normally present in the body, or that mimics a substance that is normally present in the body. Typical autoimmune diseases comprise multiple sclerosis (MS), Crohn's disease and rheumatoid arthritis.

Other diseases that are treated with interferon and for which it may be beneficial of classifying cells according to a method of the invention are malignancies such as hairy cell leukemia, malignant melanoma, and AIDS-related Kaposi's sarcoma; chronic hepatitis B and C; multiple sclerosis; condylomata acuminate, genital and perianal warts caused by infections with human papillomavirus (HPV); chronic granulomatous disease, severe, malignant osteopetrosis, chronic viral hepatitis, heamatological malignancies such as multiple myeloma, and renal cell carcinoma. Chronical viral hepatitis, heamatoligical malignancies and renal cell carcinomas are treated with IFNalpha, while MS and multiple myeloma are treated with IFNbeta.

A preferred autoimmune disease for application of a method of the invention is multiple sclerosis (MS), more preferred Relapsing Remitting MS.

It was found that in individuals suffering from, or at risk of suffering from, an autoimmune disease such as multiple sclerosis, the responsiveness of cells that are typically responsive to exposure to a type I interferon significantly differs prior to treatment. This difference is indicative for the responsiveness of the individual to treatment with said type I interferon, and can be determined by determining a level of activity of a pathway that is modulated by type 1 interferon. The samples are preferably classified as being derived from an individual with a high, low or intermediate probability of being non-responsive or responsive to treatment with said type I interferon, based on the determined level of activity.

A response to exposure to a type I interferon can be determined clinically. Several criteria are known in the art (3;4) and can be used for determining a clinical response. Preferred criteria are determining a change in Expanded Disability Status Score (EDSS); a difference in relapse rate 2 years before and 2 years after start of the therapy; and/or formation of a new T2 lesion as measured by magnetic resonance imaging. Non-responsiveness is preferably determined by an increased disability as determined by an increase in EDSS after 6 months of exposure to interferon and/or the presence of one or more relapses during exposure to interferon.

Various type I interferons exist. The most notable are the naturally occurring interferon alpha (IFNalpha) and interferon beta (IFNbeta). However, many different variants have been made that retain the activity of the original interferon. For instance, a completely artificial type I interferon was generated from the amino acid composition of interferon alpha and beta. This molecule was termed "consensus" interferon. In the present invention a molecule is said to be a type I interferon if it is interferon alpha, interferon beta or a functional part, derivative and/or analogue thereof having at least the same activity in kind as a type I interferon although the amount of activity does not necessarily need to be the same. A functional part of IFN is a part of IFN comprising the same gene activity modulating activity in kind as IFN itself. The amount of activity of such a part may differ from the activity of the complete protein. A person skilled in the art is capable of generating a suitable derivative of IFN. Derivatives can, for instance, be obtained by conservative amino acid substitution, indeed some of the currently prescribed human interferons differ slightly in amino acid sequence from natural human interferons. Examples of commercially available type I interferons are: Rebif™, a liquid form of Interferon beta 1a; Avonex™, lyophilized form of Interferon beta 1a; Cinnovex™, generic/biosimilar form of Interferon beta 1a (Avonex™); Betaseron™, Interferon beta 1b; Roferon A™, regular Interferon-alpha2a; Intron-A™, regular Interferon-alpha2b; and Pegasys™, Pegylated Interferon alpha 2a. A type I interferon of the invention may thus also be modified chemically, for instance through the addition of PEG.

A suitable part of IFN is for instance a part with an altered glycosylation pattern or a part that is non-glycosylated. Glycosylation can be prevented by removing or altering a glycosylation site of the molecule. If the generation of such a (partially) deglycosylated IFN requires alteration of the amino acid composition than such a deglycosylated IFN is derivative of a functional part of IFN. A functional part, derivative and/or analogue of IFN comprises the same activity in kind not necessarily in amount.

In general, IFN may modulate the profile of cytokine production toward that of the anti-inflammatory phenotype (for instance by upregulation of IL-10), and this appears to occur in the systemic circulation and within the CNS. All type-I interferons (interferon-alpha and interferon-beta) exert their effect through the type-I-interferon-receptor (IFN-R1). A functional part, derivative and/or analogue of IFN therefore preferably comprises the same signalling activity through IFN-R1 in kind not necessarily in amount. In the present invention interferon-beta was used to demonstrate the effectiveness of a method of the invention, however, an alternative type I interferon (for example, interferon-alpha) is also effective.

The mode of activity of a type I interferon is largely conserved in the mammalian kingdom. A rat interferon has activity on human cells. Both human and primate IFN is active in humans. In a preferred embodiment, said type I interferon is a primate type I interferon or a functional part, derivative and/or analogue thereof. In a particularly preferred embodiment said type I interferon is a human type I interferon. Preferably said type I interferon is interferon beta or a functional part, derivative, analogue and/or equivalent thereof.

Many cells are responsive for a type I interferon. With the phrase "cells that are typically responsive to exposure to a type I interferon" is meant cells of a type that, when obtained from normal (healthy) individuals, are responsive to a type I interferon, when exposed thereto in a normal amount/concentration. Non-limiting examples of such cells comprise cheek cells as present in buccal mucosal scrapings, and epithelial cells such as keratinocytes. A preferred example of a sample of cells is a sample of blood, or total blood cells. Preferably, the sample comprises peripheral blood mononuclear cells (PBMC) or a cell fraction thereof that is typically responsive to exposure to a type I interferon. Particularly preferred examples of such cells are peripheral monocytes, B cells and T cells.

A method of the invention is particularly suited for the classification of samples or cells of individuals suffering from or at risk of suffering from multiple sclerosis.

The responsiveness of an individual suffering from or at risk of suffering from multiple sclerosis towards type 1 interferon-mediated treatment is inversely related to the basal level of activity of a pathway that is modulated by type 1 interferon prior to said treatment. An increased activity of said pathway prior to treatment reduces the chance of responding to said treatment, whereby said activity is compared to the activity of reference sample comprising cells from, for example, an individual not suffering from multiple sclerosis, or an individual suffering from multiple sclerosis but being responsive to said treatment. A threshold can be set, based on data obtained from responders and non-responders. If the basal level of activity of a pathway scores above said threshold, said responsiveness can be classified as having an increased risk of being non-responsive. If the basal activity score below said threshold, said responsiveness can be classified as having an increased risk of being responsive. It will be clear to a skilled person that more than one arbitrary threshold can be set for classifying cells from a human individual depending on the derived reliability of the classification.

A level of activity of a pathway that is modulated by type 1 interferon can be measured in a number of ways known in the art. Known methods comprise determining a cellular localization or a level of phosphorylation of intermediate signalling molecules such as STAT1, STAT2 and IRF7 with antibodies and for example confocal microscopy or fluorescence-activated cell sorting, and determining serum levels of interferon-regulated cytokines such as for example, interleukin 6 and 15, CCL2 (MCP-1), CCL3, CCL8 (MCP-2), CCL19, and CXCL9, 10, and 11 using multiplex immunoassay. In a preferred embodiment said level of activity is determined by determining an expression level of at least one gene of table 2, LGALS3BP or Siglec-1 in said cells. In the present invention, the genes listed in table 2, LGALS3BP and Siglec-1 were found to be indicative of a level of activity of a pathway that is modulated by type 1 interferon, and, therefore, can be used for determining said activity.

A preferred method for determining an expression level is determining a level of RNA in said cells. In the present invention it is preferred that the level of RNA in said cells is determined for at least one gene of table 2, LGALS3BP or Siglec-1. Preferably said at least one gene that is listed in table 2 LGALS3BP or Siglec-1 has an R value of at least -0.65. Preferably the RNA levels in said cells of at least 5 and preferably at least 10, more preferably at least 15 genes of table 2 are determined. By increasing the number of genes for the determination, the accuracy of the classification increases. Preferably said at least 5, 10 or 15 genes each comprise an R value of at least -0.65 in said table. All of the 15 genes listed with an R value of at least -0.65 can be used in the present analysis. When the RNA levels of less than 5 genes are determined it is preferred that at least one of the determined RNA levels involves the level of RSAD2. When RNA levels of at least 5 genes are determined it is preferred that the RNA level of at least RSAD2 (Viperin), IFIT1 (alias G10P1; IFI56; RNM561), MX1 (alias MxA; IFI78), G1P2 (alias ISG15; IFI15), and Image: 1926927 are determined. In another preferred embodiment at least the genes or the genes products of the genes RSAD2, IFIT1, MX1, ISG15, EPSTI1, IRF7, LY6E, OAS1, OAS3, SERPING1 are measured. These provide an even better result than the already suitable first 5 of table 2. In another preferred embodiment at least the first 10 genes and or gene products of table 2 are measured (i.e. RSAD2 till LY6E). These also provide a better result than the already suitable first 5 of table 2.

Thus in a preferred embodiment said sample is a sample that has been obtained from said individual prior to initiation of the treatment with said type I interferon. The gene signatures can also be used to follow and/or determine the responsiveness of the individual once the treatment has been initiated. In this latter case it is preferred that said sample comprises a sample that is obtained from the individual both before and after initiation of the treatment with said type I interferon.

The classification in a method of the invention is preferably done by comparing the determined level of activity with a reference. The reference can be a determined level of activity of said pathway from another sample of cells of said individual. In this case it is preferred that one of said sample is collected prior to initiation of treatment with a type I interferon and another of said sample is collected after initiation of said treatment. Preferably, a method of the invention further comprises comparing said level of activity with the level of activity of said pathway in a sample of cells from said individual while receiving treatment with a type I interferon. In another preferred embodiment cells in a sample are divided into two fractions wherein the level of activity of said pathway is determined for cells of both of said fractions and wherein a first of said fractions comprising untreated cells (resting cells) and wherein a second of said fractions comprises cells that have been cultured in the presence of a type I interferon prior to determining a level of activity of said pathway, said method further comprising classifying said cells on the basis of a comparison of the level of activity in said two fractions. The untreated cells may be cells that are directly frozen after collection. The untreated cells may also be separated from serum and/or other cells in the collection sample prior to freezing. The untreated cells may also be a whole blood sample. The cells may be cultured, however, to remain "untreated" they may not be cultured together with a type I interferon. In case said sample is a protein sample it is preferred that the untreated sample is a serum sample.

In another preferred embodiment expression levels are compared between resting and type I interferon (preferably IFNbeta) treated purified PBMC. Preferably said PBMC are derived from the same sample wherein one part of said sample represents resting PBMC. Another part of said sample is cultured according to the invention in the presence of a type I interferon (preferably IFNbeta) and represents type I interferon (preferably IFNbeta) treated purified PBMC. In this embodiment it is particularly preferred that said sample is a sample from an individual that is not treated with interferon at the time of sample collection. Preferably, said individual is an individual that is being prescreened for type I interferon responsiveness, preferably for determining whether the individual is to be treated for MS with a type I interferon. It is preferred that the expression levels for the comparison are determined for the genes RSAD2, MxA and STAT1. Preferably said expression levels are determined by means of quantitative PCR, preferably by means of quantitative real-time PCR.

Expression profiles of sets of genes can be determined using a variety of methods. Methods for determining RNA expression level, such as Northern blotting, are known in the art and can be applied for the current invention. Preferred examples are quantitative amplification methods such as PCR, and methods involving the use of (micro)arrays containing probes for the respective RNAs. Preferred PCR-based methods comprise multiplex PCR and multiplex ligation-dependent probe amplification. The array format is particularly useful for this purpose. Using an array format it is possible to generate gene signatures that discriminate between individuals that have a high, low or intermediate probability to be responsive to treatment with a type I interferon.

Microarrays consist of solid support on which DNA fragments derived from individual genes are placed in an ordered array. These arrays are hybridized with fluorescent cDNA probes prepared from cellular mRNA. Two types of microarrays are most commonly used. One comprises oligonucleotides that are produced by in situ oligonucleotide synthesis using photolithographic masking techniques. In this type, genes are represented by 11 to 16 oligonucleotides

(25-mers), each including a perfect match and a mismatch that is identical except for a single base mismatch in its center. Another type of microarrays consists of longer sequences (20~2000 bp) of cDNA (PCR products) or oligonucleotides with each element representing a distinct gene that are printed on glass microscope slides (15-16).

Hybridization of the cDNA probes to microarrays results in specific base pairing with the corresponding gene sequence at known locations on the microarray. Following washing, the specific hybridization signal of the fluorescent cDNA probes to each DNA spot is quantified using a confocal scanning device. The scanned images are transformed into a gene expression matrix. Subsequently, different bioinformatics software can be applied to analyze the data. Data analysis comprises normalization of the data to reduce bias within and between experiments.

A preferred method for determining an RNA expression level comprises Taqman Low Density Arrays (TLDA; Applied Biosystems), which are preloaded customizable 384-well micro fluidic cards for target class and pathway studies based on Taqman realtime PCR. Custom-designed TLDA cards can be used to measure genes of interest. Using this high-throughput system the expression of all genes can be analyzed simultaneously for up to eight samples using minimal amounts of sample.

An alternative method for determining an expression level of at least one gene of table 2 LGALS3BP or Siglec-1 is by determining a protein expression level. Said protein expression level can be determined by any method known to a skilled person, including but not limited to Western blotting, flow cytometry, immunohistochemistry, and enzyme-linked immuno sorbent assay (ELISA). Preferred methods comprise flow cytometry and/or ELISA.

The invention further provides a kit of part comprising a set of probes or primers specific for RNA of at least 5 and preferably at least 10, more preferably at least 15 genes of table 2. Preferably wherein said at least 5, 10 or 15 genes each comprise an R value of at least -0.65 in said table.

The invention further provides the use of a kit according to the invention for classifying a sample of an individual suffering from or at risk of suffering from multiple sclerosis.

In another aspect the invention provides a method for classifying an individual as an individual with a reduced capacity to respond to type 1 beta interferon mediated therapy, said method comprising:
- determining in a sample comprising protein from said individual the level of one or more proteins or glycoproteins,
- comparing the level or levels determined with a predetermined level or levels related to a decreased response to type 1 beta interferon mediated therapy; and
- assessing, based on the comparison if the individual has a reduced capacity to respond to type 1 beta interferon mediated therapy.

In this aspect of the invention it is preferred that said sample is a sample of body fluid. Preferably a blood sample. More preferably a serum or plasma sample of said individual. In a preferred embodiment the level of a protein encoded by a gene of table 2, LGALD3BP, or Siglec-1 is determined. In a preferred embodiment the level of protein encoded by the gene LGALD3BP is determined.

The invention further provides a method for classifying an individual as an individual with a reduced capacity to respond to type 1 interferon mediated therapy, said method comprising:
- determining in a nucleic acid sample from said individual one or more polymorphisms in the IFR5 gene related to a decreased response to type 1 interferon mediated therapy, and;
- determining, based the genotype of said polymorphism(s), if the individual has a reduced capacity to respond to type 1 interferon mediated therapy.

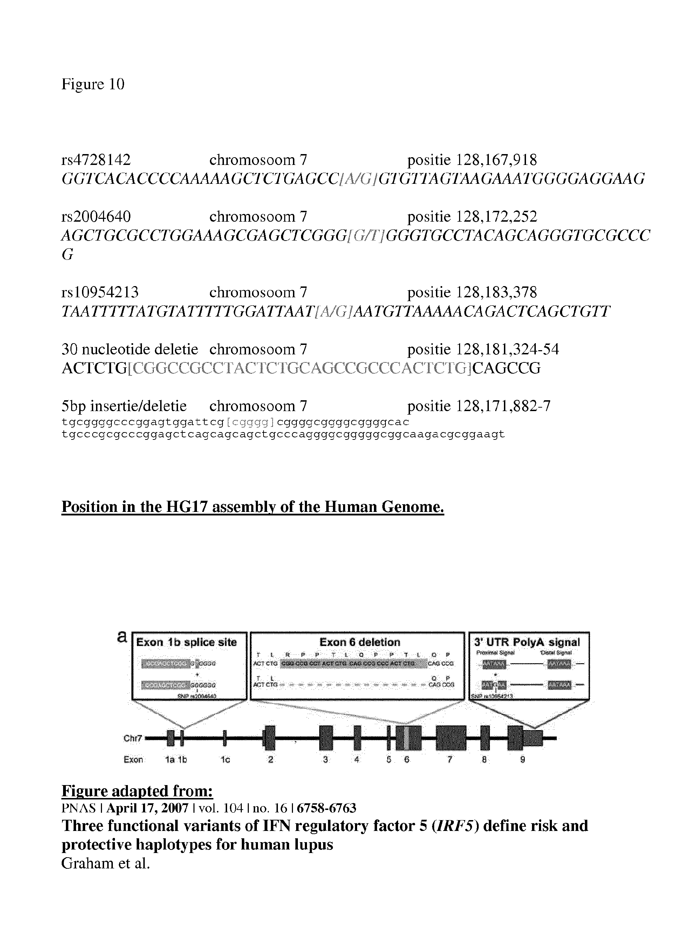

In a preferred embodiment said one or more polymorphisms comprises a polymorphism in SNP rs2004640, in SNP rs4728142, or in a 30 bp insertion-deletion polymorphism in exon 6 as depicted in figure 10.

When patients are divided in good or bad responders (or undetermined) based on clinical data (EDSS score and/or relapse rate measured during 2 years before start of therapy versus during 2 years after start of therapy) we see a high percentage of bad responders in the group of patients homozygous for the T allele of rs2004640 and/or homozygous for the A allele of rs4728142.

The TT genotype of rs2004640 is associated with low/bad biological response. The TT genotype of rs2004640 is associated with low/bad clinical response. The AA genotype of rs4728142 is associated with low/bad biological response. The AA genotype of rs4728142 is associated with low/bad clinical response. The TT genotype of rs2004640 is associated with high baseline levels of the IFN response genes of the gene set.

The AA genotype of rs4728142 is associated with high baseline levels of the IFN response genes of the gene set.

Patients homozygous for the 5bp CGGGG deletion show a low/bad biological response.

Patients homozygous for the 5bp CGGGG deletion show a bad clinical response.

In a preferred embodiment more than one polymorphism is determined to indicate whether an individual is a good or bad responder to treatment with a type I interferon. It is preferred to determine the haplotype for the indicated polymorphisms and classify the cells of the individual on the basis of of the determined haplotype. In this embodiment Haplotype 1 (rs4728142 (A) rs2004640 (T) exon 6 indel (del) rs10954213 (A)) associated with a bad/low biological response and Haplotype 8 (rs4728142 (G) rs2004640 (G) exon 6 indel (in) rs10954213 (G)) is associated with a good/high biological response. Thus in one aspect the invention provides the use a polymorphism as indicated in figure 10 for determining whether an individual is likely to be a good, bad or normal responder to treatment with a type I interferon. In a preferred embodiment said polymorphism is a polymorphism of rs4728142, rs2004640, exon 6 indel (del) or exon 6 indel (in), or rs10954213 as indicated in figure 11. In a preferred embodiment the haplotype for at least two and preferably at least 3 and more preferably all of the polymorphisms mentioned are determined. The polymorphism is also determined according to the invention if the complementary strand is analysed. In this case, the correlations and predictions as indicated herein above, are of course associated with the presence of the respective complementary nucleotide(s). The combining feature is that these polymorphisms are all situated in or close by the IRF5 gene. As this gene is likely to show more polymorphic sites the artisan is capable of finding further polymorphic sites that correlate well with the indicated polymorphisms. Thus the present invention further provides a method for classifying an individual as an individual with a reduced capacity to respond to type 1 interferon mediated therapy, said method comprising:
- determining in a nucleic acid sample from said individual a further polymorphic site associated with the IFR5 gene and determining whether said further polymorphic site exhibits one or more polymorphism that correlate for more than 90% with a polymorphism as indicated in figure 10.

The invention further provides the use of a polymorphism that discriminates alleles of the IFR5 gene and that correlates for more than 90% with a polymorphism associated with a poor or with a good responder to treatment with a type I interferon as indicated in figure 10 and herein above, for classifying an individual as an individual with a reduced, a normal or a good capacity to respond to type 1 interferon mediated therapy.

The invention further provides a kit comprising a set of probes or PCR primers specific for the detection of polymorphism in SNP rs2004640, SNP rs4728142, or a 30 bp insertion-deletion polymorphism in exon 6 of the IFR5 or a polymorphism associated with the IFR5 gene that correlates for more than 90% with a polymorphism associated with a poor or with a good responder to treatment with a type I interferon as indicated in figure 10 and herein above, for use in a method according to the invention.

The invention further provides a method for classifying cells from a human individual said method comprising:
providing a sample comprising cells from said individual that are typically responsive to exposure to a type I interferon;
determining a level of activity of a pathway that is modulated by type 1 interferon; and
classifying said cells on the basis of the determined level of activity. Preferably the method further comprises culturing said cells in the presence of a type I interferon prior to determining a level of activity of said pathway. Said individual is preferably suffering from or at risk of suffering from an autoimmune disease. Said autoimmune disease is preferably multiple sclerosis.

Preferably said classification comprises classifying said cells as from an individual with a reduced capacity to respond to type 1 interferon-mediated therapy.

Said type I interferon is preferably interferon-1B or an equivalent thereof. Preferably said level of activity of a pathway that is modulated by type 1 interferon is determined by determining an expression level of at least one gene of table 2, LGALS3BP or Siglec-1 in said cells. Preferably said expression level is determined by determining a level of RNA in said cells. Preferably said at least one gene comprises an R value of at least -0.65 in said table. Preferably said method comprises determining the RNA levels in said cells of at least 5 and preferably at least 10, more preferably at least 15 genes of table 2. Preferably said at least 5, 10 or 15 genes each comprises an R value of at least -0.65 in said table. Preferably said at least one gene comprises RSAD2. Preferably said at least 5 genes comprise at least RSAD2, IFIT1, MX1, G1P2, and Image: 1926927.

Preferably said RNA level is measured by means of an array.

Preferably said expression level is determined by determining a protein expression level. Preferably said expression level is determined by flow cytometry and/or ELISA.

Preferably said sample comprises peripheral blood mononuclear cells or a cell fraction thereof that is typically responsive to exposure to a type I interferon. Preferably said classification further comprises comparing the determined level of activity with a reference.

Preferably said sample is derived from an individual prior to treatment with a type I interferon. Preferably the method further comprises comparing said level of activity with the level of activity of said pathway in a sample of cells from said individual while receiving treatment with a type I interferon. Preferably cells in a sample are divided into two fractions, wherein the level of activity of said pathway is determined for cells of both of said fractions and wherein a first of said fractions comprising untreated cells (resting cells) and wherein a second of said fractions comprises cells that have been cultured in the presence of a type I interferon prior to determining a level of activity of said pathway, said method further comprising classifying said cells on the basis of a comparison of the level of activity in said two fractions.

Also provided is a kit of part comprising a set of probes or primers specific for RNA of at least 5 and preferably at least 10, more preferably at least 15 genes of table 2. Preferably said at least 5, 10 or 15 genes each comprise an R value of at least -0.65 in said table. Also provided is the use of the kit for classifying a sample of an individual suffering from, or at risk of suffering from, multiple sclerosis.

Also provided is a method for classifying an individual as an individual with a reduced capacity to respond to type 1 beta interferon mediated therapy, said method comprising:
a. determining in a sample comprising protein from said individual the level of one or more proteins or glycoproteins
b. comparing the level or levels determined in step a with a predetermined level or levels related to a decreased response to type 1 interferon mediated therapy
c. assessing, based on the comparison of step b, if the individual has a reduced capacity to respond to type 1 beta interferon mediated therapy. Preferably the protein is a product of the LGALS3BP gene.

Also provided is a method for classifying an individual as an individual with a reduced capacity to respond to type 1 interferon mediated therapy, said method comprising:
- determining in a nucleic acid sample from said individual one or more polymorphisms associated with the IFR5 gene and related to a type of response to type 1 interferon mediated therapy,
- determining, based the genotype of said polymorphism(s), if the individual has a reduced, normal and/or good capacity to respond to type 1 interferon mediated therapy. Preferably the one or more polymorphisms comprises the SNP rs2004640, the SNP rs4728142, and/or the 30 bp insertion-deletion polymorphism in exon 6 as indicated in figure 10.

Also provided is a kit comprising a set of probes or PCR primers specific for the detection of polymorphism in SNP rs2004640, SNP rs4728142, and the 30 bp insertion-deletion polymorphism in exon 6 of the IFR5 gene as indicated in figure 10 for use in a method for classifying an individual as an individual with a reduced capacity to respond to type 1 interferon mediated therapy, said method comprising:
- determining in a nucleic acid sample from said individual one or more polymorphisms associated with the IFR5 gene and related to a type of response to type 1 interferon mediated therapy,
- determining, based the genotype of said polymorphism(s), if the individual has a reduced, normal and/or good capacity to respond to type 1 interferon mediated therapy, wherein the one or more polymorphisms comprises the SNP rs2004640, the SNP rs4728142, and/or the 30 bp insertion-deletion polymorphism in exon 6 as indicated in figure 10

### Brief description of the drawings

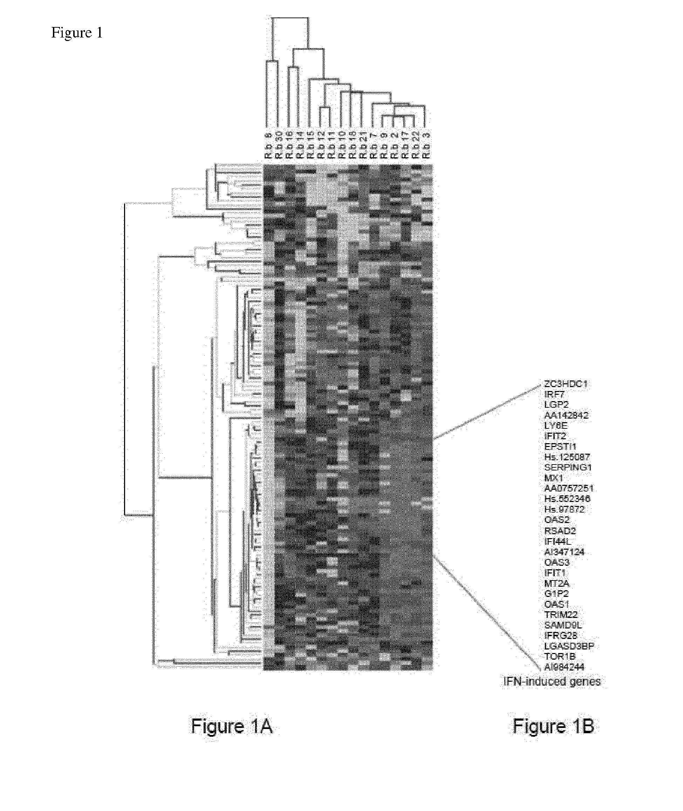

Figure 1A; Biological response to IFNbeta therapy in MS patients Treeview diagram representing genes that show at least a two-fold difference in gene expression ratio (biological response) relative to median expression ratio after a two-way hierarchical Cluster analysis without centering the gene expression ratios. Upregulated genes after therapy are indicated by a red colour, downregulated by a green colour and genes that show no differences in expression after therapy are indicated in black.
Figure 1B; Cluster of IFN-induced genes. Selection of genes clustering together based on similar biological response profiles within the patient group. The genes cluster together with a correlation of 0.925 and are known to be induced by IFN. The mean expression ratio of all genes in this IFN cluster is referred as the biological IFN-response.

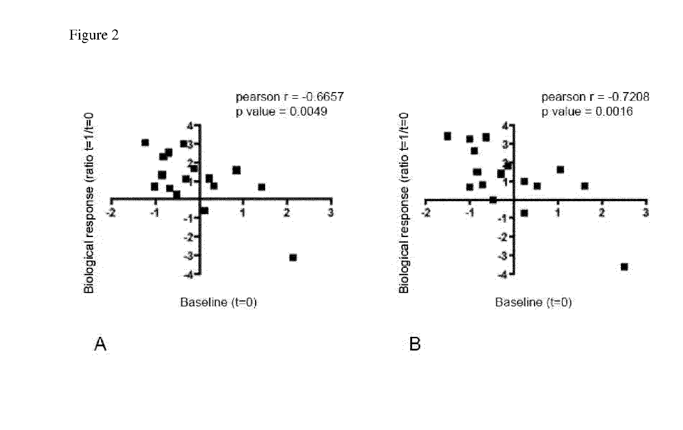

Figure 2; Correlation between baseline gene expression levels and biological response to IFNbeta therapy Mean expression levels on baseline and mean biological response of a set of IFN-induced genes were calculated and are correlated with each other, resulting in a significant negative correlation; A IFN cluster as described in Figure 1B; B selection of 15 genes.

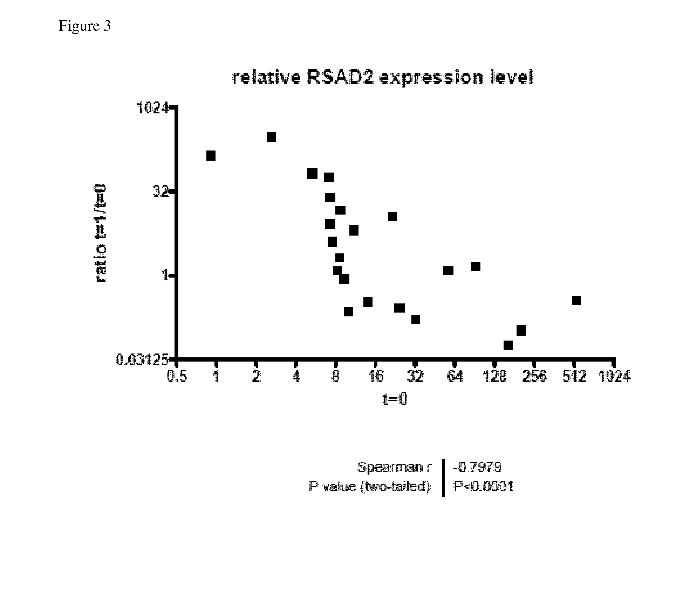

Figure 3; Correlation between baseline RSAD2 gene expression level and biological response to IFNbeta therapy in a group of 22 individuals. A relative baseline expression level of RSAD2 as measured by quantitative real-time PCR is indicated on the X-axis. The y-axes indicates a biological response (gene expression level of RSAD2 before start of IFNbeta therapy divided by gene expression level of RSAD2 after start (=during) of IFNbeta therapy.

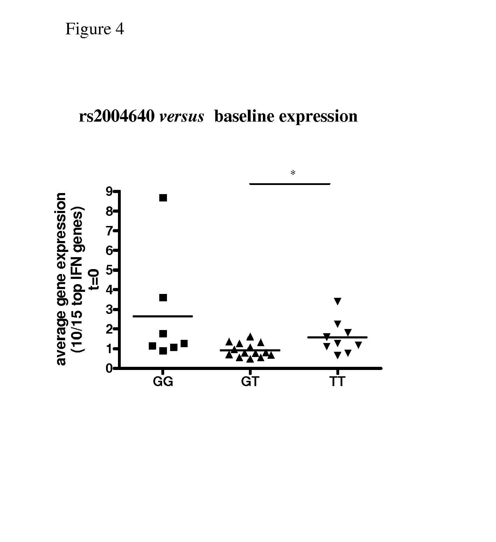

Figure 4; Mean gene expression level of a set of the top ten 10 IFN type I response genes of table 2 (RSAD2, IFIT1, MX1, ISG15, EPSTI1, IRF7, LY6E, OAS1, OAS3, SERPING1) in patients with genotype GG, GT and TT of the SNP rs 2004640 at baseline. Patients homozygous for the T allel have a significant higher baseline IFN type I response gene expression (P=0.0198) than heterozygous patients. The TT genotype of rs2004640 is associated with high baseline levels of the IFN response genes of the gene set.

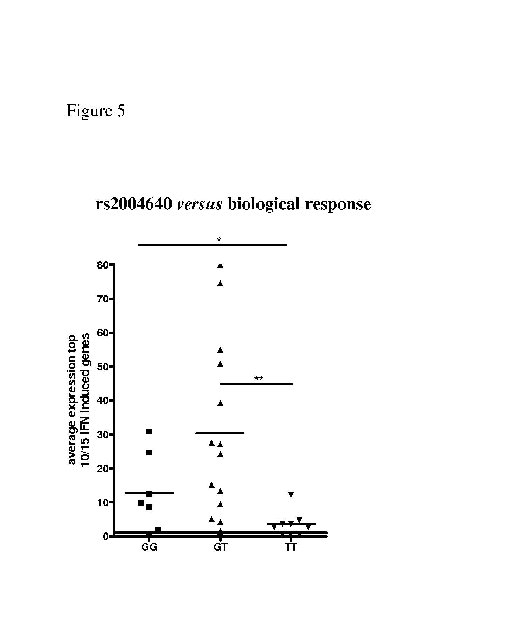

Figure 5; Mean gene expression level of a set of the top ten 10 IFN type I response genes of table 2 (RSAD2, IFIT1, MX1, ISG15, EPSTI1, IRF7, LY6E, OAS1, OAS3, SERPING1) with genotype GG, GT and TT of the SNP rs 2004640 after pharmacological intervention with IFN-b. A significant reduced biological response was observed for patients homozygous for the T allel versus heterozygous patients (P=0.0057) and patients homozygous for the G allel (0.0340)

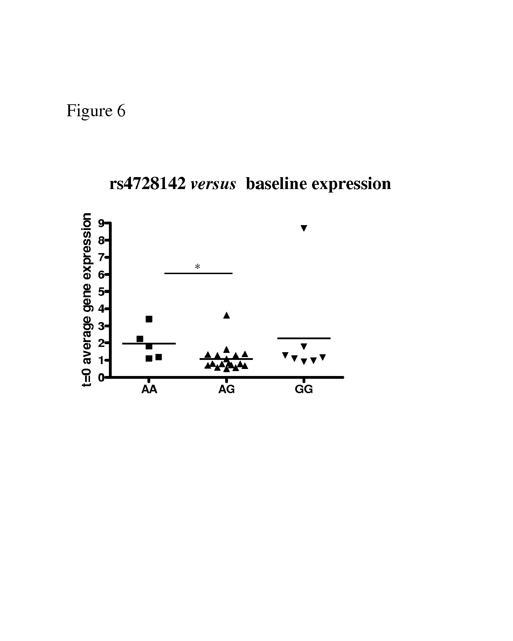

Figure 6; Mean gene expression level of a set of the top ten 10 IFN type I response genes of table 2 (RSAD2, IFIT1, MX1, ISG15, EPSTI1, IRF7, LY6E, OAS1, OAS3, SERPING1) in patients with genotype GG, GT and TT of the SNP rs4728142 at baseline. Patients homozygous for the A allel have a significant higher baseline IFN type I response gene expression (P=0.0394) than heterozygous patients.

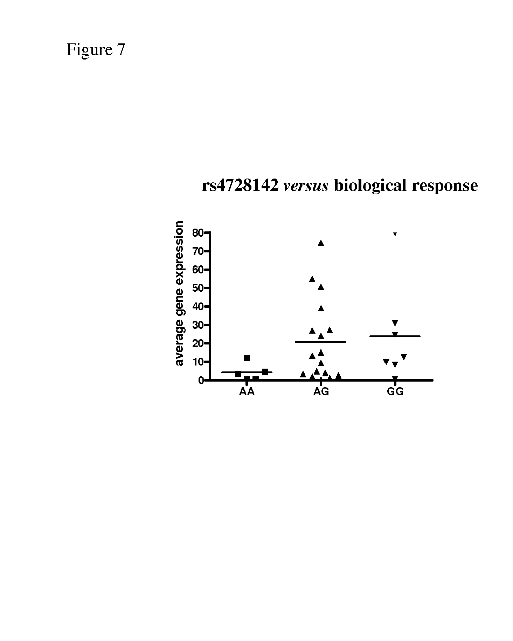

Figure 7; Mean gene expression level of a set of the top ten 10 IFN type I response genes of table 2 (RSAD2, IFIT1, MX1, ISG15, EPSTI1, IRF7, LY6E, OAS1, OAS3, SERPING1) in patients with genotype GG, GT and TT of the SNP rs4728142 after pharmacological intervention with IFN-b. Patients homozygous for the A allele have a lower biological response than heterozygous patients (p=0.1198) and homozygous for the G allele (p=0.1421)

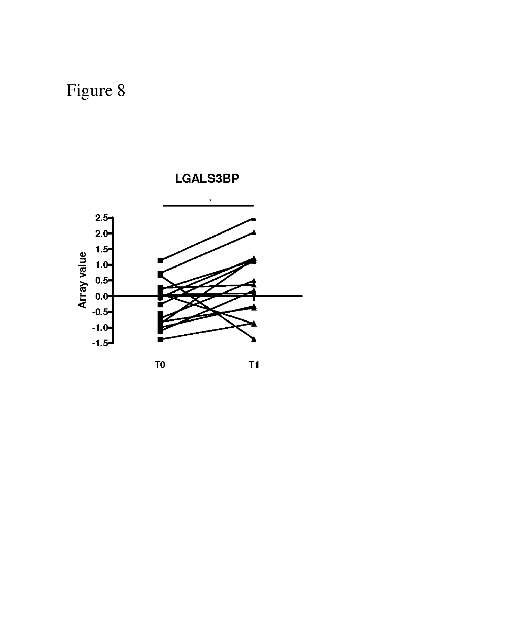

Figure 8; The expression level of LGALS3BP of 15 MS patients before and after treatment with IFN beta shows that in most patient expression is upregulated after treatment.

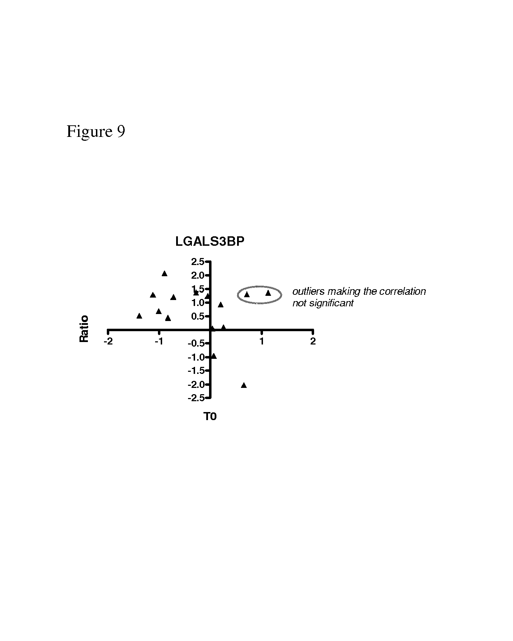

Figure 9; Correlation between baseline LGALS3BP gene expression level and biological response to IFN beta therapy in a group of 15 individuals. The relative baseline expression level of LGALS3BP is indicated on the X-axis. The y-axes indicates a biological response (gene expression level of LGALS3BP after (=during) start of IFNbeta therapy divided by gene expression level of LGALS3BP before start of IFNbeta therapy.
Figure 10: characteristics of polymorphisms

### Examples

### Example 1 (see also: van Baarsen LG, Vosslamber et al., PLoS ONE. 2008) Patients

A group of 16 Dutch patients (10 females and 6 males) with clinically definite relapsing remitting MS was recruited from the outpatient clinic of the MS Centre Amsterdam. Mean age at start of IFNbeta therapy is 40.6 ± 7.7, mean EDSS is 2.3 ± 1.3 (range 1-6). Blood samples were obtained just before treatment and 1 month after start of the therapy. Patients received either Avonex (n=4), Betaferon (n=7), Rebif 22 (=2) or Rebif 44 (n=3).

### Blood sampling

From each patient blood was drawn into one PAXgene tube (PreAnalytix, GmbH, Germany) and three heparin tubes (Beckton Dickinson, Alphen a/d Rijn, Netherlands). After blood collection, tubes were transferred from the clinic to the lab within one hour in order to isolate fresh peripheral blood mononuclear cells (PBMCs) from the heparin tubes using lymphoprep (Axis-Shield, Lucron) density gradient centrifugation. PAXgene tubes were stored at room temperature (RT) for two hours to ensure complete lyses of all blood cells after which tubes were stored at -20 until RNA isolation. Total RNA was isolated within 7 months after storage. Tubes were thawed 2 hours at RT prior to RNA isolation. Next, RNA was isolated using the PreAnalytix RNA isolation kit according to the manufacturers' instructions, including a DNAse (Qiagen) step to remove genomic DNA. Quantity and quality of the RNA was tested using the Nanodrop spectrophotometer (Nanodrop Technologies, Wilmington, Delaware USA)o the manufacturers' instructions, including a DNAse (Qiagen) step to remove genomic DNA. Quantity and quality of the RNA was tested using the Nanodrop spectrophotometer (Nanodrop Technologies, Wilmington, Delaware USA).

### Microarray hybridisation

We used 43K cDNA microarrays from the Stanford Functional Genomics Facility (http://microarray.org/sfgf/) printed on aminosilane-coated slides containing ~17,000 unique genes. First DNA spots were UV-crosslinked to the slide using 150-300 mJoules. Prior to sample hybridisation, slides were prehybridised at 42 degrees Celsius for 15 minutes in a solution containing 40% ultrapure formamide (Invitrogen, Breda, Netherlands), 5% SSC (Biochemika, Sigma), 0.1% SDS (Fluka Chemie, GmbH, Switserland) and 50 µg/ml BSA (Panvera, Madison, USA). After prehybridisation slides were briefly rinsed in MilliQ water, thoroughly washed in boiling water and 95% ethanol and air-dried. Sample preparation and microarray hybridisation was performed as described previously (17), apart from the different postprocessing and prehybridisation described above.

### Microarray analysis

Image analysis and data filtering was performed using the Stanford Microarray Database (18) (http://genome-www5.stanford.edu//) as described previously (19). Statistical Analysis of Microarrays (20) (SAM) was used to determine significantly differential expressed genes. A gene was considered as significantly differential expressed if the False Discovery Rate (FDR) was equal or less than 5%. Cluster analysis (21) was used to define clusters of co-ordinately changed genes after which the data was visualized using Treeview. Realtime PCR.

RNA (0.5 µg) was reverse transcribed into cDNA using a Revertaid H-minus cDNA synthesis kit (MBI Fermentas, St. Leon-Rot, Germany) according to the manufacturers' instructions. Quatative realtime PCR was performed using an ABI Prism 7900HT Sequence detection system (Applied Biosystems, Foster City, CA, USA) using SybrGreen (Applied Biosystems). Primers were designed using Primer Express software and guidelines (Applied Biosystems) and are listed in table 4. To calculate arbitrary values of mRNA levels and to correct for differences in primer efficiencies a standard curve was constructed. Expression levels of target genes were standardized against housekeeping gene glyceraldehydes-3-phosphate dehydrogenase (GAPDH), parallel detected in the identical cDNA samples.

### In vitro study

Freshly isolated PBMCs were washed using PBS containing 1% fetal calf serum (FCS; BioWhittaker, Cambrex) and plated in 24-wells culture plates at a density of 2x106 cells per ml per well. Cells were stimulated or not with 10 Units recombinant IFNbeta (Abcam, Cambridge, UK) for 4h after which RNA was isolated using the Rneasy Qiagen RNA isolation kit (Qiagen, Venlo, Netherlands) according to the manufacturers' instructions. A DNAse (Qiagen) step was included to remove genomic DNA. Quantity and quality of the RNA was tested using the Nanodrop spectrophotometer (Nanodrop Technologies, Wilmington, Delaware USA)

### Statistical analysis

Correlation analyses were performed using Graphpad Prism 4 software. First, data was tested for normal distribution. In case data passes normality test, correlation was tested using Pearson correlation. Spearman correlation was used in case of nonparametric distribution of the data. Correlation was considered significant if p-values were less than 0,05.

### Example 2 (see also: van Baarsen LG, Vosslamber et al., PLoS ONE. 2008) Pharmacogenomics of IFNbeta therapy in MS

In order to understand the pharmacodynamics of IFNbeta therapy we analysed the peripheral blood gene expression profiles of 16 RRMS patients before and one month after the start of therapy. Two class paired analysis using Significant Analysis of Microarrays (SAM) (False Discovery Rate (FDR) <5%) between pre- and post-therapy data was applied to identify genes that differed significantly. Surprisingly, only 3 genes, "Interferon alpha-inducible protein 27" (IFI27), "Ring finger protein 36" (RNF36) and "Epithelial stromal interaction protein 1 (breast)" (EPSTI1), were identified as significantly differential expressed. Hence, we conclude that the biological response to IFNbeta is low or neglectable in the MS patient population as a whole. This conclusion is consistent with findings of a suboptimal type I IFN signalling in MS (22).

Given the heterogeneous nature of MS we questioned whether the observed poor in vivo response upon IFNbeta treatment of the whole MS cohort could be a reflection of averaging out differences in pharmacodynamic responsiveness between the patients. To test this hypothesis we investigated the biological response at the individual patient level and calculated for each patient and for each gene the ratio of gene expression at pre- vs. post therapy (log-2 ratios). Two-way hierarchical (unsupervised) cluster analysis was used to select genes that differed in IFNbeta induced expression between patients. This analysis showed a marked variation in biological response to IFNbeta between patients. A total of 126 genes showed at least a two-fold difference in gene expression ratio in at least seven patients (Figure 1A). This analysis revealed that some patients showed upregulated genes, whereas in other patients the same genes are downregulated or unchanged after IFNbeta therapy. As anticipated, part of this gene expression pattern is consistent with expression of known IFN-response genes (14) (figure 1B). A number of 28 IFN-induced genes clustered tightly together (r=0.925) indicating a related function of these genes, but showed different differential relative response ratio's between patients. The expression data of some of the IFN-induced genes was validated by real time-PCR and showed a good correlation with the micro array data (Table 1A). These findings confirm the hypothesis that there exists considerable variation in the pharmacological effects of IFNbeta between patients with RRMS.

Example 3 (see also: van Baarsen LG, Vosslamber et al., PLoS ONE. 2008) Relation between differential pharmacodynamics of IFNbeta therapy and baseline expression of IFN-response genes.

Previously, we demonstrated significant differences in the expression of type I IFN-induced genes between untreated RRMS patients (19). Here we investigated whether there exists a relationship between the differential in vivo responsiveness to IFNbeta and baseline expression levels of IFN-induced genes. Therefore, we tested for each patient whether there is an association between the expression level of the type I IFN-response gene cluster (shown in Figure 1B) before therapy with the response ratio after therapy. This analysis demonstrated that the mean baseline expression of the 28 type I IFN genes negatively correlates with the in vivo IFN-induced response levels (p = 0.0049 and Pearson r = -0.6657) (Figure 2A).

In order to create a gene set that best predicts the pharmacological response to IFNbeta we selected those genes whose expression shows best negative correlation between baseline and biological response (p <0.01 and Pearson r <-0.65) (Table 2). To exclude a potential bias of the gene selection at baseline, we analyzed the correlation of the mean biological response of the selected 15 IFN-induced genes with the baseline values of all genes on the array. This resulted in three additional genes (IFI44L, MT1E and IMAGE:1879725; Pearson r <0.65 and variance >1.00) that significantly correlate with a pharmacodynamic response to IFNbeta therapy. Although these genes do not cluster tightly together with the previously selected genes, they may be important in pharmacodynamics to IFNbeta.

### Example 4 (see also: van Baarsen LG, Vosslamber et al., PLoS ONE. 2008) IFNbeta pharmacodynamics using purified PBMC

To confirm that the observed pharmacodynamic differences were due to differential responsiveness of peripheral blood cells and not because of low exposure of IFNbeta due to the presence of inhibitory plasma proteins such as neutralizing antibodies, we measured the expression of a selected set of three known IFNbeta response genes and IFNbeta itself in resting and IFNbeta treated purified PBMCs by quantitative real-time PCR. The selected IFNbeta response genes are i, RSAD2, which shows the most significant correlation of biological response versus baseline at single gene level (Table 2); ii, MxA, which shows a good negative correlation and is known as a marker of IFN bioactivity (23); and iii, STAT1, which is one of the components that is important for IFNbeta signaling. We hypothesized that baseline expression levels of IFNbeta influences subsequent IFNbeta signaling upon treatment. When comparing the in vitro biological response of these genes to the mean in vivo biological response of the selection of 15 genes a significant association was revealed (Table 3). From these results we conclude that the differential IFNbeta responsiveness in MS is a consequence of pharmacodynamic differences of the peripheral blood cells.

These data clearly show that there is evidence for differences in IFNbeta pharmacodynamics between patients with MS. Until now part of the unresponsiveness to IFNbeta was explained by the appearance of persistent neutralizing antibodies (NAbs) which were shown to be associated with a reduction in the efficacy of IFNs (24;25). It is unlikely that Nabs would be present in patients prior to baseline, since Nabs appear over time. Accordingly, we observed that the differential IFN response pattern was also apparent in PBMC from MS patients isolated before IFNbeta treatment. The in vitro response differences were consistent with the in vivo response results. Hence our findings indicate that the differential response to IFNbeta in MS is a consequence of pathophysiological differences between patients, and excludes the possibility of involvement of neutralizing antibodies to explain the defective response observed in a subset of patients.

These differences between RRMS patients demonstrate the physiopathological importance of the IFN response baseline levels as the predominating pathway that discriminates between biological responders and non-responders. We assigned a cluster of genes known to be IFN type I response genes (top 15 listed in table 2).

Example 5 (see also: van Baarsen LG, Vosslamber et al., PLoS ONE. 2008) RSDA2 gene expression in whole blood from 22 RRMS patients before and after IFNbeta therapy.

As previously described, baseline expression levels of a number of IFN-induced genes may be predictive for the biological response (= ratio of gene expression before/after treatment) to IFNbeta treatment in RRMS patients. In this pilot study we collected whole blood from a second group of 22 RRMS patients before and after the start of IFNbeta treatment.

As we observed in the previous study, gene expression levels of RSAD2 (among others) may predict the biological response to IFNbeta treatment. In this second group of patients we measured the expression of RSAD2 using quantitative realtime PCR.

In patients with a high expression of RSAD2 before start of the treatment, the expression level is low after/during treatment whereas in the patients with low expression of RSAD2 before treatment the expression clearly raises after/during IFNbeta treatment (see Figure 3). This negative correlation between baseline levels and gene expression levels of RSAD2 after/during treatment confirms the result of the previous study.

### Example 6

To determine biological response, peripheral blood was collected from 30 RRMS patients before and during IFN-b therapy. From 20 untreated RRMS patients peripheral blood was collected at two time points over a three to twelve month time period to analyze the stability of baseline values over time. Baseline stability and biological response rate, were analyzed by Taqman Low Density Arrays (TLDA) using the mean gene expression level of a set of the top 10 IFN type I response genes of table 2 (RSAD2, IFIT1, MX1, ISG15, EPSTI1, IRF7, LY6E, OAS1, OAS3, SERPING1). Genetic variation was determined the IRF5 gene, a component of the IFN signaling cascade. A panel of three (rs2004640, rs10954213, rs4728142) Single Nucleotide Polymorphisms (SNP) and one 30 bp insertion-deletion polymorphism in exon 6 were genotyped. The SNPs were genotyped using Taqman Genotyping Assay (Applied Biosystems). The 30bp indel was amplified as a 115/145 bp fragment using conventional PCR. The PCR-amplified fragments were separated on a 2.5% agarose gel and visualized using ethidium bromide staining.

The extent of the biological response correlated negatively with the baseline expression of the type I IFN response gene set (R=-0.3891; p=0.0336). The baseline stability over time was reflected by a correlation efficient of r=0.54; P=0.029 (n=20).

Next we determined the association of three SNPs and the 30 bp insertion-deletion polymorphism in the IRF5 gene with IFN type I response gene activity at baseline and after pharmacological intervention with IFN-beta. For rs2004640 we showed that patients homozygous for the T allel have a significant higher baseline IFN type I response gene expression (P=0.0198) than heterozygous patients (figure 4). Accordingly, a significant reduced biological response was observed for patients homozygous for the T allel versus heterozygous patients (P=0.0057) and patients homozygous for the G allel (0.0340) (figure 5). For rs4728142, patients homozygous for the A allel have a significant higher baseline IFN type I response gene expression (P=0.0394) than heterozygous patients (figure 6) and a trends towards a lower biological response than heterozygous patients (p=0.1198) and homozygous for the G allel (p=0.1421) (figure 7).

We performed haplotype analysis and determined that haplotype 1 is associated with bad/low biological response and haplotype 8 is associated with good/high biological response.
Haplotype 1: rs4728142 (A) rs2004640 (T) exon 6 indel (del) rs10954213 (A)
Haplotype 8: rs4728142 (G) rs2004640 (G) exon 6 indel (in) rs10954213 (G)

When patients are divided in good or bad responders (or undetermined) based on clinical data (EDSS score and/or relapse rate measured during 2 years before start of therapy versus during 2 years after start of therapy) we see a high percentage of bad responders in the group of patients homozygous for the T allele of rs2004640 and/or homozygous for the A allele of rs4728142.

### Conclusions:

- The TT genotype of rs2004640 is associated with low/bad biological response.
- The TT genotype of rs2004640 is associated with low/bad clinical response.
- The AA genotype of rs4728142 is associated with low/bad biological response.
- The AA genotype of rs4728142 is associated with low/bad clinical response.
- The TT genotype of rs2004640 is associated with high baseline levels of the IFN response genes of the gene set.
- The AA genotype of rs4728142 is associated with high baseline levels of the IFN response genes of the gene set.
- Patients homozygous for the 5bp CGGGG deletion show a low/bad biological response.
- Patients homozygous for the 5bp CGGGG deletion show a bad clinical response.

### Example 7 LGALS3BP (alias protein 90K/Mac-2BP) gene expression in whole blood from 15 MS patients before and after IFN beta therapy

As previously described, baseline expression levels of a number of IFN-induced genes may be predictive for the biological response (= ratio of gene expression before/after treatment) to IFN beta treatment in MS patients. In this pilot study we collected whole blood from a group of 15 MS patients before and after the start of IFN beta treatment.

As we observed in the previous study, gene expression levels of LGALS3BP (among others) may predict the biological response to IFNbeta treatment. In patients with a high expression of LGALS3BP before start of the treatment, the expression level is low after/during treatment whereas in the patients with low expression of LGALS3BP before treatment the expression clearly raises after/during IFN beta treatment (see Figure 8 and Figure 9). Therefore, elevated serum levels of LGALS3BP are a predictive biomarker for failure to respond to type I IFN treatment, as has been shown previously for interferon-alpha treated patients with chronic hepatitis C infection (Artini M et al., Hepatol. 1996 Aug;25(2):212-7).

### Example 8

### Siglec-1 expressed on monocytes in peripheral blood as a biomarker for response to IFNb therapy

Sialic acid-binding Ig-like lectin 1 (Siglec-1, sialoadhesin, CD169)) is known as one of the most prominent type I IFN-regulated candidate genes. Biesen et al. (Arthr. Rheum. 2008 Apr;58(4):1136-45) demonstrated that Siglec-1 expression in inflammatory and resident monocytes is a potential biomarker for monitoring disease activity and success of therapy in systemic lupus erythematosus. Levels of Siglec-1 were determined using multicolor flow cytometry. It was shown that the frequency of Siglec-1-expressing monocyte subsets was correlated with disease activity (as measured by the SLE Disease

Activity Index) and was inversely correlated with levels of complement factors. Most interestingly, levels of anti-double-stranded DNA (anti-dsDNA) antibodies were highly correlated with the percentage of resident monocytes, but not inflammatory monocytes, expressing Siglec-1. High-dose glucocorticoid treatment resulted in a dramatic reduction of Siglec-1 expression in cells from patients with active SLE. Thus Siglec-1 expression in resident blood monocytes is a potential biomarker for type I IFN responses that are indicative for disease activity. Therefore, levels of Siglec-1 expressed on monocytes in MS and other IFN related diseases (melanona, Hepatitis C infection) can be used as a biomarker for baseline type I IFN response avtivity, that is predictive for the response to treatment.

### References

(1) Hafler DA, Slavik JM, Anderson DE, O'Connor KC, De JP, Baecher-Allan C. Multiple sclerosis. Immunol Rev 2005 April;204:208-31.
(2) Interferon beta-1b in the treatment of multiple sclerosis: final outcome of the randomized controlled trial. The IFNB Multiple Sclerosis Study Group and The University of British Columbia MS/MRI Analysis Group. Neurology 1995 July;45(7):1277-85.
(3) Rudick RA, Lee JC, Simon J, Ransohoff RM, Fisher E. Defining interferon beta response status in multiple sclerosis patients. Ann Neurol 2004 October;56(4):548-55.
(4) Rio J, Nos C, Tintore M, Tellez n, Galan I, Pelavo R, Comabella M, Montalban X. Defining the response to interferon-beta in relapsing remitting multiple sclerosis patients. Ann Neurol 2006 February;59(2):344-52
(5) Reske D, Walser A, Haupt WF, Petereit HF. Long-term persisting interferon beta-1b neutralizing antibodies after discontinuation of treatment. Acta Neurol Scand 2004 January;109(1):66-70.
(6) Rice GP, Paszner B, Oger J, Lesaux J, Paty D, Ebers G. The evolution of neutralizing antibodies in multiple sclerosis patients treated with interferon beta-1b. Neurology 1999 April 12;52(6):1277-9.
(7) Sibley WA, Bamford CR, Clark K. Clinical viral infections and multiple sclerosis. Lancet 1985 June 8;1(8441):1313-5.
(8) Vartanian TK, Zamvil SS, Fox E, Sorensen PS. Neutralizing antibodies to disease-modifying agents in the treatment of multiple sclerosis. Neurology 2004 December 14;63(11 Suppl 5):S42-S49.
(9) Ghislain JJ, Fish EN. Application of genomic DNA affinity chromatography identifies multiple interferon-alpha-regulated Stat2 complexes. J Biol Chem 1996 May 24;271(21):12408-13.
(10) Li X, Leung S, Qureshi S, Darnell JE, Jr., Stark GR. Formation of STAT1-STAT2 heterodimers and their role in the activation of IRF-1 gene transcription by interferon-alpha. J Biol Chem 1996 March 8;271(10):5790-4.
(11) Brierley MM, Fish EN. Functional relevance of the conserved DNA-binding domain of STAT2. J Biol Chem 2005 April 1;280(13):13029-36.
(12) Ghislain JJ, Wong T, Nguyen M, Fish EN. The interferon-inducible Stat2:Stat1 heterodimer preferentially binds in vitro to a consensus element found in the promoters of a subset of interferon-stimulated genes. J Interferon Cytokine Res 2001 June;21(6):379-88.
(13) Takaoka A, Taniguchi T. New aspects of IFN-alpha/beta signalling in immunity, oncogenesis and bone metabolism. Cancer Sci 2003 May;94(5):405-11.
(14) Der SD, Zhou A, Williams BR, Silverman RH. Identification of genes differentially regulated by interferon alpha, beta, or gamma using oligonucleotide arrays. Proc Natl Acad Sci U S A 1998 December 22;95(26):15623-8.
(15)Brown, P. O. & Botstein, D. Exploring the new world of the genome with DNA microarrays. Nat Genet 1999; 21:33-37.
(16) 3. Hughes, T. R., Mao, M., Jones, A. R., Burchard, J., Marton, M. J., Shannon, K. W.,Lefkowitz, S. M., Ziman, M., Schelter, J. M., Meyer, M. R., Kobayashi, S., Davis, C.,Dai, H., He, Y. D., Stephaniants, S. B., Cavet, G., Walker, W. L., West, A., Coffey, E.,Shoemaker, D. D., Stoughton, R., Blanchard, A. P., Friend, S. H., & Linsley, P. S. Expression profiling using microarrays fabricated by an ink-jet oligonucleotide synthesizer. Nat. Biotechnol.2001; 19:342-347.
(17) van der Pouw Kraan TC, Baarsen EGM, Rustenburg F, Baltus B, Fero M, Verweij CL. Gene expression profiling in rheumatology. In: Cope AP, editor. Arthritis Research. Methods and Protocols, Volume 2 ed. The Humana Press Inc.; 2007.
(18) Ball CA, Awad IA, Demeter J, Gollub J, Hebert JM, Hernandez-Boussard T et al. The Stanford Microarray Database accommodates additional microarray platforms and data formats. Nucleic Acids Res 2005 January 1;33(Database issue):D580-D582.
(19) van Baarsen LG, van der Pouw Kraan TC, Kragt JJ, Baggen JM, Rustenburg F, Hooper T et al. A subtype of multiple sclerosis defined by an activated immune defense program. Genes Immun 2006 September;7(6):522-31.
(20) Tusher VG, Tibshirani R, Chu G. Significance analysis of microarrays applied to the ionizing radiation response. Proc Natl Acad Sci U S A 2001 April 24;98(9):5116-21.
(21) Eisen MB, Spellman PT, Brown PO, Botstein D. Cluster analysis and display of genome-wide expression patterns. Proc Natl Acad Sci U S A 1998 December 8;95(25):14863-8.
(22) Feng X, Petraglia AL, Chen M, Byskosh PV, Boos MD, Reder AT. Low expression of interferon-stimulated genes in active multiple sclerosis is linked to subnormal phosphorylation of STAT1. J Neuroimmunol 2002 August;129(1-2):205-15.
(23) Kracke A, von WP, Al-Masri AN, Dalley G, Windhagen A, Heidenreich F. Mx proteins in blood leukocytes for monitoring interferon beta-1b therapy in patients with MS. Neurology 2000 January 11;54(1):193-9.
(24) Li DK, Zhao GJ, Paty DW. Randomized controlled trial of interferon-beta-1a in secondary progressive MS: MRI results. Neurology 2001 June 12;56(11):1505-13.
(25) Rudick RA, Simonian NA, Alam JA, Campion M, Scaramucci JO, Jones W et al. Incidence and significance of neutralizing antibodies to interferon beta-1a in multiple sclerosis. Multiple Sclerosis Collaborative Research Group (MSCRG). Neurology 1998 May;50(5):1266-72.

Deguez-Gonzalez R, Calaza M, Perez-Pampin E, de la Serna AR, Fernandez-Gutierrez B, Castaneda S et al. Association of interferon regulatory factor 5 haplotypes, similar to that found in systemic lupus erythematosus, in a large subgroup of patients with rheumatoid arthritis. Arthritis Rheum 2008; 58(5):1264-1274.

Demirci FY, Manzi S, Ramsey-Goldman R, Minster RL, Kenney M, Shaw PS et al. Association of a common interferon regulatory factor 5 (IRF5) variant with increased risk of systemic lupus erythematosus (SLE). Ann Hum Genet 2007; 71(Pt 3):308-311.

Graham RR, Kyogoku C, Sigurdsson S, Vlasova IA, Davies LR, Baechler EC et al. Three functional variants of IFN regulatory factor 5 (IRF5) define risk and protective haplotypes for human lupus. Proc Natl Acad Sci U S A 2007; 104(16):6758-6763.

Kristjansdottir G, Sandling JK, Bonetti A, Roos IM, Milani L, Wang C et al. Interferon Regulatory Factor 5 (IRF5) Gene Variants are Associated with Multiple Sclerosis in Three Distinct Populations. J Med Genet 2008.

Sigurdsson S, Goring HH, Kristjansdottir G, Milani L, Nordmark G, Sandling JK et al. Comprehensive evaluation of the genetic variants of interferon regulatory factor 5 (IRF5) reveals a novel 5 bp length polymorphism as strong risk factor for systemic lupus erythematosus. Hum Mol Genet 2008; 17(6):872-881.

### Tables

**Table 1; C orrelation between microarray data and realtime PCR data.**

| **Genes** | **P value** | **R value** |
|---|---|---|
| MxA | 0.0188 | 0.4335 |
| OAS1 | <0.0001 | 0.6972 |
| STAT1 | <0.0001 | 0.7371 |
| RSAD2 | <0.0001 | 0.7086 |
| IRF7 | 0.0014 | 0.5648 |
| ISG15 | <0.0001 | 0.7051 |

**Table 2; Correlations of baseline vs biological responses at single gene level for the IFN cluster**

| **Gene symbol** | **R value** | **P value** |
|---|---|---|
| RSAD2 | -0 7983 | 0 0011 |
| IFTT1 | -0 7746 | 0 0004 |
| MX1 | -0 7619 | 0 0006 |
| ISG15 | -0 7532 | 0 0008 |
| IMAGE 1926927 | -0 7168 | 0 0026 |
| EPSTI1 | -0 7162 | 0 0059 |
| Transcribed locus | -0 6977 | 0 0038 |
| IRF7 | -0 6925 | 0 0029 |
| IMAGE 545138 | -0 6881 | 0 0065 |
| LY6E | -0 6834 | 0 0035 |
| OAS1 | -0 6822 | 0 0051 |
| OAS3 | -0 6787 | 0 0076 |
| IMAGE 504372 | -0 6707 | 0 0087 |
| SERPING1 | -0 6688 | 0 0064 |
| Transcribed locus | -0.6677 | 0 0047 |
| IFI44L | -0.6353 | 0.0196 |
| Transcribed locus | -0 6175 | 0 0108 |
| MT2A | -0 6166 | 0 011 |
| TRIM22 | -0.6115 | 0.0118 |
| SAMD9L | -0 6102 | 0 0121 |
| IMAGE. 2562181 | -0.591 | 0 0203 |
| OAS2 | -0 5865 | 0.0169 |
| LGP2 | -0 5842 | 0 0222 |
| ZC3HDC1 | -0 5482 | 0 0343 |
| TOR1B | -0 4914 | 0 0532 |
| IFTT2 | -0 4426 | 0 086 |
| IFRG28 | -0 369 | 0 1759 |
| LGALS3BP | -0 279 | 0.314 |

**Table 3; Correlation between biological resp on se of single IFN-induced genes in vitro versus mean biological response in vivo of 15 genes**

| Genes | P value | R. value |
|---|---|---|
| RSAD2 | 0 0012 | 0.7518 |
| MxA | 0 0280 | 0 6064 |
| STAT1 | 0.0100 | 0.6614 |
| IFNb | 0 0036 | 0 7675 |

**Table 4; Primers used for realtime PCR**

| Genes | Genebank accession nr. | Sense primer | Antisense primer | Length PCR product (bp) |
|---|---|---|---|---|
| MxA | NM 002462 | TTCAGCACCTGATGGCCTATC | GTACGTC TGGAGCATGAAGAAC TG | 92 |
| OAS1 | NM 016816 | TGCGCTCAGCTTCGTACTGA | G GTG GA GAAC TC GCCCTCTT | |
| STAT1 | NM 007315 | TGC ATC ATG GGC TTC ATC AGC | GAA GTC AGG TTC GCC TCC GTT C | 156 |
| RSAD2 | NM 080657 | GTGGTTCCAGAA TTATGGTGAGTATTT | CCACGGCCAATAAGGACATT | 90 |
| IRF7 | NM 004031 | GCTCCCCACCCTATACCATCTAC | GCCAGGGTTCCAGCTTCAC | 99 |
| ISG15 | NM 005101 | TTTGCCAGTACAGGAGCTTGTG | GGGTGA TCTGCGCCTTCA | 151 |
| IFNb | NM 002176 | ACAGACTTACAGGTTACCTCCGAAAC | CTCCTACCCTCTCCCTCTGGGACTGG | 93 |

## Claims

1. A method for classifying cells from a human individual suffering from or at risk of suffering from an interferon treatable disease, preferably an interferon treatable autoimmune disease, said method comprising:
providing a sample comprising cells from said individual that are typically responsive to exposure to a type I interferon;
determining a basal level of activity of a pathway that is modulated
by type 1 interferon; and
classifying said cells on the basis of the determined basal level of activity, preferably, wherein said classification further comprises comparing the determined basal level of activity with a reference.

2. A method for classifying cells from a human individual suffering from or at risk of suffering from an interferon treatable disease, preferably an interferon treatable autoimmune disease said method comprising:
providing a sample comprising cells from said individual that are typically responsive to exposure to a type I interferon;
determining a level of activity of a pathway that is modulated by
type 1 interferon; and
classifying said cells on the basis of the determined level of activity, preferably wherein said level of activity of a pathway that is modulated by type 1 interferon is determined by determining an expression level of RSAD2.

3. A method according to claim 2, wherein cells in a sample are divided into two fractions, wherein the level of activity of said pathway is determined for cells of both of said fractions and wherein a first of said fractions comprising untreated cells (resting cells) and wherein a second of said fractions comprises cells that have been cultured in the presence of a type I interferon prior to determining a level of activity of said pathway, said method further comprising classifying said cells on the basis of a comparison of the level of activity in said two fractions.

4. A method according to any one of claims 1-3, said sample is derived from an individual prior to treatment with a type I interferon.

5. A method according to any one of claims 1-4, wherein said autoimmune disease is multiple sclerosis or rheumatoid arthritis.

6. A method according to any one of claims 1-4, wherein said level of activity is determined by measuring the level of IFN-beta.

7. A method according to any one of the previous claims, wherein said classification comprises classifying said cells as from an individual with a reduced capacity to respond to type 1 interferon-mediated therapy.

8. A method according to any of the previous claims, wherein said level of activity of a pathway that is modulated by type 1 interferon is determined by determining an expression level of at least one gene of table 2, LGALS3BP or Siglec-1 in said cells, preferably wherein said at least one gene comprises RSAD2.

9. A method according to claim 8, wherein said expression level is determined by determining a level of RNA in said cells and wherein said at least one gene comprises an R value of at least -0.65 in said table.

10. A method according to claim 9, comprising determining the RNA levels in said cells of at least 5, preferably wherein said at least 5 genes comprise at least RSAD2, IFIT1, MX1, G1P2, and Image: 1926927; preferably at least 10, more preferably at least 15 genes of table 2, preferably wherein said at least 5, 10 or 15 genes each comprises an R value of at least -0.65 in said table.

11. A method for classifying cells from a human individual suffering from or at risk of suffering from an interferon treatable disease, preferably an interferon treated autoimmune disease said method comprising:
providing a sample comprising cells from said individual that are typically responsive to exposure to a type I interferon;
determining the level of IFN-beta; and classifying said cells on the basis of the determined level.

12. A method according to any one of the previous claims, wherein said sample comprises peripheral blood mononuclear cells or a cell fraction thereof that is typically responsive to exposure to a type I interferon.

13. A kit of part comprising a set of probes or primers specific for RNA of at least 5, preferably at least 10, more preferably at least 15 genes of table 2.

14. Use of a kit according to claim 13, for classifying cells from a human individual suffering from or at risk of suffering from an interferon treatable disease, preferably an interferon treatable autoimmune disease.

15. A method for classifying an individual as an individual with a reduced capacity to respond to type 1 beta interferon mediated therapy, said method comprising:
a. determining in a sample comprising protein from said individual the level of one or more proteins or glycoproteins, preferably, wherein the protein is a product of the LGALS3BP gene
b. comparing the level or levels determined in step a with a predetermined level or levels related to a decreased response to type 1 interferon mediated therapy
c. assessing, based on the comparison of step b, if the individual has a reduced capacity to respond to type 1 beta interferon mediated therapy.


SEQUENCE LISTING

<110> Vereniging VU-Windesheim Vereniging voor Christelijk Hoger
      Onderwijs, Wetenschappelijk Onderzoek en Patiëntenzorg
      Polman, Chris H
      van Baarsen, Elisabeth G.M.
      Stahlecker-Voslamber, Saskia
      Verweij, Cornelis L

<120> Means and methods for classifying samples of multiple sclerosis
      patients

<130> P81368EP30

<140> EP 12167621.7
<141> 2012-05-11

<150> EP07109468.4
<151> 2007-06-01

<150> EP10169971.8
<151> 2008-06-02

<160> 25

<170> PatentIn version 3.3

<210> 1
<211> 20
<212> DNA
<213> Artificial

<220>
<223> primer

<400> 1
ttcgcacctg atggcctatc                                                  20

<210> 2
<211> 24
<212> DNA
<213> Artificial

<220>
<223> antisense primer

<400> 2
gtacgtctgg agcatgaaga actg                                             24

<210> 3
<211> 20
<212> DNA
<213> Artificial

<220>
<223> primer

<400> 3
tgcgctcagc ttcgtactga                                                  20

<210> 4
<211> 20
<212> DNA
<213> Artificial

<220>
<223> anti-sense primer

<400> 4
ggtggagaac tcgccctctt 20


<210> 5
<211> 21
<212> DNA
<213> Artificial

<220>
<223> primer

<400> 5
tgcatcatgg gcttcatcag c 21


<210> 6
<211> 22
<212> DNA
<213> Artificial

<220>
<223> anti-sense primer

<400> 6
gaagtcaggt tcgcctccgt tc 22


<210> 7
<211> 27
<212> DNA
<213> Artificial

<220>
<223> primer

<400> 7
gtggttccag aattatggtg agtattt 27


<210> 8
<211> 20
<212> DNA
<213> Artificial

<220>
<223> anti-sense primer

<400> 8
ccacggccaa taaggacatt 20

<210> 9
<211> 23
<212> DNA
<213> Artificial

<220>
<223> primer

<400> 9
gctccccacg ctataccatc tac 23


<210> 10
<211> 19
<212> DNA
<213> Artificial

<220>
<223> anti-sense primer

<400> 10
gccagggttc cagcttcac 19


<210> 11
<211> 22
<212> DNA
<213> Artificial

<220>
<223> primer

<400> 11
tttgccagta caggagcttg tg 22


<210> 12
<211> 18
<212> DNA
<213> Artificial

<220>
<223> anti-sense primer

<400> 12
gggtgatctg cgccttca 18


<210> 13
<211> 26
<212> DNA
<213> Artificial

<220>
<223> primer

<400> 13
acagacttac aggttacctc cgaaac 26

<210>  14
<211>  26
<212>  DNA
<213>  Artificial

<220>
<223>  anti-sense primer

<400>  14
ctcctagcct gtccctctgg gactgg                                          26


<210>  15
<211>  52
<212>  DNA
<213>  Homo sapiens


<220>
<221>  variation
<222>  (27)..(27)
<223>  g

<400>  15
ggtcacaccc caaaaagctc tgagccagtg ttagtaagaa atggggagga ag             52


<210>  16
<211>  52
<212>  DNA
<213>  Homo sapiens


<220>
<221>  variation
<222>  (27)..(27)
<223>  t

<400>  16
agctgcgcct ggaaagcgag ctcggggggg tgcctacagc agggtgcgcc cg              52


<210>  17
<211>  52
<212>  DNA
<213>  Homo sapiens


<220>
<221>  variation
<222>  (27)..(27)
<223>  g

<400>  17
taatttttat gtatttttgg attaataaat gttaaaaaca gactcagctg tt             52


<210>  18
<211>  42
<212>  DNA
<213>  Homo sapiens

<400>  18
actctgcggc cgcctactct gcagccgccc actctgcagc cg                          42


<210>  19
<211>  12
<212>  DNA
<213>  Homo sapiens

<400>  19
actctgcagc cg                                                           12


<210>  20
<211>  105
<212>  DNA
<213>  Homo sapiens

<400>  20
tgcggggccc ggagtggatt cgcggggcgg ggcggggcgg ggcactgccc gcgcccggag      60

ctcagcagca gctgcccagg ggcgggggcg gcaagacgcg gaagt                      105


<210>  21
<211>  100
<212>  DNA
<213>  Homo sapiens

<400>  21
tgcggggccc ggagtggatt cgcggggcgg ggcggggcac tgcccgcgcc cggagctcag      60

cagcagctgc ccaggggcgg gggcggcaag acgcggaagt                            100


<210>  22
<211>  16
<212>  DNA
<213>  Homo sapiens

<400>  22
gcgagctcgg gtgggg                                                       16


<210>  23
<211>  16
<212>  DNA
<213>  Homo sapiens

<400>  23
gcgagctcgg gggggg                                                       16


<210>  24
<211>  42
<212>  DNA
<213>  Homo sapiens

<400>  24
actctgcggc cgcctactct gcagccgccc actctgcagc cg                          42

<210> 25
<211> 12
<212> DNA
<213> Homo sapiens

<400> 25
actctgcagc cg
12